(19) Europäisches Patentamt
European Patent Office
Office européen des brevets

(11) EP 0 585 170 B1

(12) FASCICULE DE BREVET EUROPEEN

(45) Date de publication et mention de la délivrance du brevet:
**06.11.1996 Bulletin 1996/45**

(51) Int Cl.[6]: **C07C 65/40**, C07C 65/10, A61K 7/48, A61K 31/19

(21) Numéro de dépôt: **93402077.7**

(22) Date de dépôt: **20.08.1993**

# (54) Composition pour le traitement de l'acné contenant au moins un dérivé d'acide salicylique

Mindestens ein Salicylsäurederivat enthaltende Zusammensetzung für die Behandlung von Akne

Composition for treating acne which contains at least one derivative of salicyclic acid

(84) Etats contractants désignés:
**AT BE CH DE DK ES FR GB GR IE IT LI NL PT SE**

(30) Priorité: **24.08.1992 FR 9210227**

(43) Date de publication de la demande:
**02.03.1994 Bulletin 1994/09**

(73) Titulaire: **L'OREAL**
**75008 Paris (FR)**

(72) Inventeurs:
- **Sebag, Henri**
**F-75016 Paris (FR)**
- **Ribier, Alain**
**F-75014 Paris (FR)**
- **Simon, Pascal**
**F-94400 Vitry-sur-Seine (FR)**
- **Sebillotte, Laurence**
**F-75013 Paris (FR)**

(74) Mandataire: **Peuscet, Jacques**
**SCP Cabinet Peuscet et Autres,**
**68, rue d'Hauteville**
**75010 Paris (FR)**

(56) Documents cités:
**EP-A- 0 124 905**
**EP-A- 0 317 846**
**FR-A- 2 607 498**
**US-A- 4 514 385**

EP 0 585 170 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne une composition pour le traitement de l'acné comportant un dérivé d'acide salicylique et des dérivés d'acide salicylique particuliers utilisables dans ladite composition.

L'étiopathogénie de l'acné, bien que mal définie, prend son origine dans la formation d'une lésion caractéristique, le comédon. Celui-ci résulte de l'obstruction du canal pilosébacé par suite d'une diskératinisation de la zone de l'infundibilum du canal. Cette obstruction a pour effet majeur de modifier la rhéologie du sébum et les caractéristiques physico-chimiques du milieu, tels que pH et tension de vapeur d'oxygène. Cette modification permet l'hyperprolifération des souches résidentes cutanées, principalement du PROPIONI-BACTERIUM ACNES, qui est une souche anaérobie ou aéro-tolérante. L'hyperprolifération bactérienne a pour conséquence de libérer dans le milieu certaines protéases ou hyaluronidases, d'origine bactérienne, qui provoquent une lyse du sac folliculaire et la libération de composés inflammatoires au sein du derme, déclenchant ainsi une réaction de type inflammatoire de l'organisme. Si la nature des composés inflammatoires est à l'heure actuelle non déterminée, leur origine bactérienne semble faire peu de doute, expliquant le bon succès thérapeutique dans l'acné inflammatoire de composés antibactériens tant par voie orale que par voie topique.

On a déjà proposé d'utiliser, à titre d'agents antibactériens, pour le traitement de l'acné des dérivés de l'acide salicylique, notamment dans FR-A 2 581 542 et FR-A 2 607 498. Ces agents antibactériens sont utilisés sous forme d'émulsions, de gels ou de lotions.

On sait, par ailleurs, que certains lipides amphiphiles possèdent la propriété de former des phases mésomorphes, dont l'état d'organisation est intermédiaire entre l'état cristallin et l'état liquide et que, parmi eux, certains sont susceptibles de gonfler en présence d'une solution aqueuse pour former une phase lamellaire puis, après agitation, former des vésicules dispersées dans une phase aqueuse de dispersion référencée "D". Ces vésicules sont formées par une membrane constituée par des feuillets sensiblement concentriques, composés d'une ou plusieurs couches multimoléculaires, de préférence bimoléculaires, les différentes couches de lipide étant séparées par une couche de phase aqueuse et encapsulant une phase aqueuse encapsulée référencée "E". Les lipides amphiphiles susceptibles de former des vésicules sont, de façon connue, des lipides amphiphiles ioniques et/ou des lipides amphiphiles non-ioniques.

Ces vésicules peuvent être préparées par de nombreux procédés. Selon un premier procédé, qui est par exemple décrit par BANGHAM et al. (J. Mol. Bio., 13, 1965 pages 238 à 262), on dissout la phase lipidique dans un solvant volatil, on forme un film mince de phase lipidique sur les parois d'un flacon par évaporation du solvant, on introduit la phase à encapsuler sur le film lipidique et on agite le mélange mécaniquement jusqu'à obtention de la dispersion de vésicules ayant la taille désirée ; on obtient ainsi une dispersion aqueuse de vésicules encapsulant une phase aqueuse E, la phase aqueuse encapsulée E et la phase aqueuse de dispersion D étant identiques. Selon un second procédé, dit par cofusion de lipides, décrit par exemple dans FR-A- 2 315 991, on prépare la phase lipidique par mélange du (des) lipide (s) amphiphile(s) et des éventuels additifs, à une température où le mélange est fondu, si le mélange n'est pas liquide à température ambiante ; on forme une phase lamellaire, par introduction de la phase aqueuse à encapsuler E ; puis on disperse la phase lamellaire sous forme de vésicules, à l'aide d'un ultra-disperseur, d'un homogénéiseur ou d'ultrasons, dans une phase aqueuse de dispersion D. Dans une variante de ce procédé, la formation de la phase lamellaire ne constitue pas un stade séparé du procédé. Les vésicules obtenues par ces deux procédés sont généralement de type "multifeuillet". Pour obtenir des vésicules de type "monofeuillet", on peut utiliser un procédé qui consiste à solubiliser le mélange lipidique par l'octylglucoside et à former les vésicules selon l'enseignement de FR-A 2 543 018. Cette énumération de procédés n'est aucunement limitative et l'invention est applicable quel que soit le procédé de fabrication des vésicules sous forme de dispersion dans une phase aqueuse.

Il est connu d'introduire dans la phase lipidique des additifs destinés à améliorer la stabilité physicochimique (floculation, fusion), à diminuer la perméabilité de la membrane lipidique aux substances encapsulées, et à augmenter le taux d'encapsulation.

Les additifs destinés à améliorer la stabilité physico-chimique sont, de façon connue, des stérols et des lipides chargés. Les stérols, en particulier le cholestérol, ont pour rôle d'étendre le domaine de stabilité des vésicules sur une plus grande plage de température et de diminuer leur perméabilité aux substances encapsulées dans la phase aqueuse d'encapsulation.

Les lipides chargés, tels que le dicétyl ou le dimyristyl phosphate, le cholestérylphosphate, l'acide phosphatidique, les amines ou ammonium quaternaire à longues chaînes, ont pour rôle d'améliorer la stabilité des vésicules en prévenant leur floculation et, par conséquent, leur fusion, même en présence d'électrolytes, et de permettre l'augmentation du taux d'encapsulation de substances hydrosolubles en accroissant l'épaisseur des couches aqueuses qui séparent les feuillets lipidiques constituant la membrane. Il est connu d'ajouter à la phase lipidique plusieurs de ces additifs, en particulier au moins un additif du type stérol et au moins un additif du type lipide chargé.

Il est, par ailleurs, également connu d'incorporer dans la phase lipidique un actif cosmétique et/ou dermopharmaceutique. Les actifs utilisables doivent être liposolubles et doivent ne pas détruire la stabilité physico-chimique des vésicules, ne pas augmenter de façon gênante leur perméabilité aux substances encapsulées, et ne pas diminuer le

taux d'encapsulation.

Certains composés, par exemple le cholestérol, améliorent la stabilité physico-chimique et le taux d'encapsulation et ont également une activité cosmétique et/ou dermopharmaceutique . On utilise donc, de préférence, des composés qui ont une activité cosmétique et/ou dermopharmaceutique et qui, de plus, améliorent la stabilité des vésicules et leur taux d'encapsulation.

Selon la présente invention, on a découvert que certains dérivés de l'acide salicylique, qui ont une activité anti-acné, peuvent également être incorporés comme lipides chargés anioniques dans la phase lipidique formant la membrane de vésicules de lipides amphiphiles. On peut donc ainsi obtenir des vésicules qui ont une bonne stabilité physico-chimique, une perméabilité convenable, un bon taux d'encapsulation et qui ont également une activité anti-acné.

L'invention a donc pour objet une composition pour le traitement de l'acné par application topique contenant une dispersion , dans une phase aqueuse D, de vésicules de lipide(s) amphiphile(s) constituées par une membrane de phase lipidique encapsulant une phase aqueuse E, la phase lipidique contenant comme additif un lipide chargé caractérisé par le fait que la phase lipidique contient au moins un dérivé d'acide salicylique de formule :

I 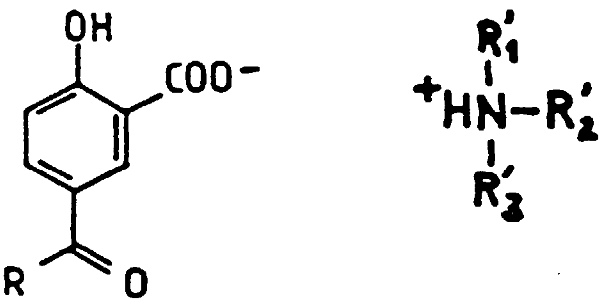

formule (I) dans laquelle :

- R représente un radical alkyle linéaire ou ramifié en $C_{11}$ - $C_{17}$, de préférence, un radical alkyle linéaire en $C_{11}$,
- $R'_1$, $R'_2$ et $R'_3$ identiques ou différents, représentent un radical alkyle ou hydroxyalkyle en $C_1$ - $C_{18}$, un des radicaux $R'_1$, $R'_2$ ou $R'_3$ pouvant être un radical benzyle.

La phase lipidique contient de préférence de 3 % à 10% en poids de composé de formule (I). Pour des quantités inférieures à 3 % les vésicules n'ont pas d'activité antiacné et pour des quantités supérieures à 10% la stabilité physico-chimique des vésicules obtenues commence à diminuer.

Le dérivé d'acide salicylique de formule (I) préféré est le n-dodécanoyl-5 salicylate de N, N-diméthyl N-(hydroxy-2 éthyl) ammonium.

Il n'était pas évident que l'incorporation en quantité allant jusqu'à 10% de dérivés d'acide salicylique de formule (I) dans la phase lipidique formant la membrane des vésicules, permettrait d'obtenir des vésicules ayant une bonne stabilité physico-chimique et un bon taux d'encapsulation. En effet des essais ont montré qu'on ne peut pas introduire plus de 4% d'acide dodécanoyl-5 salicylique dans la phase lipidique de lipides amphiphiles. En effet pour des proportions supérieures ce dérivé précipite dans la phase lipidique. L'incorporation de l'octanoyl-5 salicylate d'hexadécyl pyridinium ou de dodecanoyl-5 salicylate d'hexadécyl triméthylammonium n'améliore pas la stabilité physico-chimique des vésicules et il est donc nécessaire d'introduire dans la phase lipidique un lipide chargé conventionnel. Le dodécyl-5 salicylate de triméthyl β-hydroxy éthylammonium incorporé à un taux allant jusqu'à 10% en poids permet d'obtenir des vésicules ayant une bonne stabilité physico-chimique mais la capacité d'encapsulation des vésicules diminue d'environ 50% par rapport à la capacité d'encapsulation des vésicules préparées avec les mêmes lipides mais ne contenant pas ledit dodécyl salicylate.

Par contre on a constaté que, dans une phase lipidique déterminée, contenant du dicetylphosphate, fréquemment utilisé comme lipide chargé, on pouvait remplacer la totalité du dicetylphosphate par un dérivé de l'acide salicylique de formule (I) selon l'invention sans modifier pratiquement la stabilité physico-chimique des vésicules obtenues et leur taux d'encapsulation.

Selon l'invention la phase lipidique constitutive des membranes des vésicules de la dispersion comprend de façon connue, au moins un lipide amphiphile choisi dans le groupe formé par :

A/les lipides non-ioniques ci-après définis :

(1) les dérivés du glycérol, linéaires ou ramifiés, de formule :

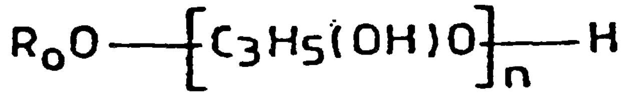

formule dans laquelle :

. $C_3H_5(OH)O$-
représente les structures suivantes prises en mélange ou séparément :

$$-CH_2CHOHCH_2O-,$$

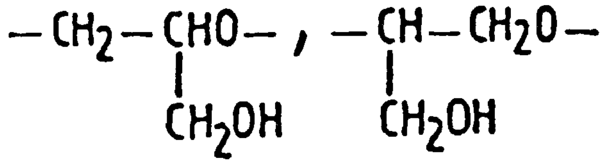

. n est une valeur statistique moyenne comprise entre 1 et 6 ou bien n est une valeur réelle égale à 1 ou 2, auquel cas $C_3H_5(OH)O$ représente la structure $-CH_2CHOH-CH_2O-$ ;

. $R_o$ représente :

(a)une chaîne aliphatique, linéaire ou ramifiée, saturée ou insaturée, contenant de 12 à 30 atomes de carbone ; ou des radicaux hydrocarbonés des alcools de lanoline ; ou les restes d'alpha-diols à longue chaîne;
(b)un reste $R_1CO$, où $R_1$ est un radical aliphatique, linéaire ou ramifié, en $C_{11}$-$C_{29}$ ;
(c)un reste :

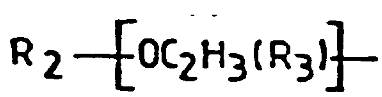

où

. $R_2$ peut prendre la signification (a) ou (b) donnée pour $R_o$;

$$-OC_2H_3(R_3)-$$

représente les structures suivantes, prises en mélange ou séparément :

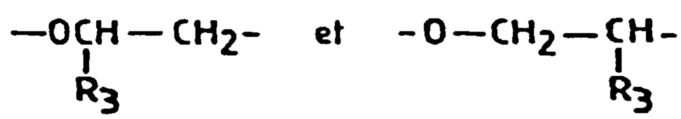

où

- $R_3$ prend la signification (a) donnée pour $R_o$ ;

(2)les éthers de polyglycérol, linéaires ou ramifiés, comportant deux chaînes grasses ;
(3)les diols à chaîne grasse ;
(4)les alcools gras oxyéthylénés ou non, les stérols tels que, par exemple, le cholestérol et les phytostérols,

oxyéthylénés ou non ;
(5)les éthers et esters de polyols, oxyéthylénés ou non, l'enchaînement des oxydes d'éthylène pouvant être linéaire ou cyclique ;
(6)les glycolipides d'origine naturelle ou synthétique, les éthers et esters de mono ou polysaccharides et notamment les éthers et les esters de glucose ;
(7)les hydroxyamides décrits dans le brevet français N° 2 588 256 et représentés par la formule :

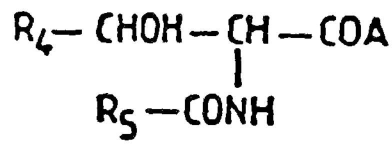

formule dans laquelle :

- $R_4$ désigne un radical alkyle ou alcényle en $C_7$-$C_{21}$ ;
- $R_5$ désigne un radical hydrocarboné, saturé ou insaturé, en $C_7$-$C_{31}$ ;
- COA désigne un groupement choisi parmi les deux groupements suivants :

  . un reste

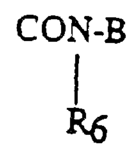

  où :
  . B est un radical alkyle dérivé d'amines primaires ou secondaires, mono ou polyhydroxylées ; et
  . $R_6$ désigne un atome d'hydrogène ou un radical méthyle, éthyle ou hydroxyéthyle ; et
  . un reste, -COOZ, où Z représente le reste d'un polyol en $C_3$-$C_7$.

(8)les céramides naturels ou de synthèse ;
(9)les dihydroxyalkylamines, les amines grasses oxyéthylénées ;
(10)les dérivés du glycérol décrits dans la demande de brevet WO- A 92/08685 et répondant à la formule :

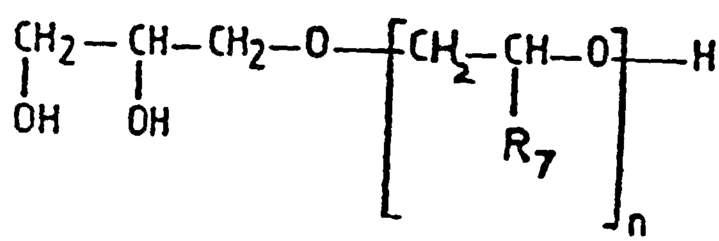

formule dans laquelle $R_7$ représente un radical alkyle linéaire en $C_{14}$ - $C_{18}$ ou un groupement $-CH_2A$ dans lequel A est $OR_{14}$, $R_{14}$ représentant un radical alkyle linéaire en $C_{10}$-$C_{18}$ et, de préférence, en $C_{16}$, et n représente une valeur statistique moyenne supérieure à 1 et au plus égale à 3 et, en outre, lorsque $R_7$ =$-CH_2A$, n peut également représenter une valeur réelle (non statistique) égale à 2.
(11)les esters de glucose de formule :

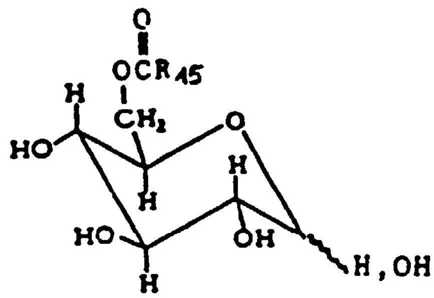

formule dans laquelle $R_{15}$ représente une chaîne hydrocarbonée en $C_9$-$C_{17}$ linéaire, saturée ou insaturée, décrits dans EP-A 485251.

B)les lipides amphiphiles ioniques ci-après définis :

(1) les lipides amphiphiles anioniques tels que :

- . les phospholipides naturels, notamment la lécithine d'oeuf ou de soja, ou la sphingomyéline, les phospholipides modifiés par voie chimique ou enzymatique, notamment la lécithine hydrogénée, et les phospholipides de synthèse, notamment la dipalmitoylphosphatidylcholine ;
- . des composés anioniques, tels que ceux décrits dans le brevet français N° 2 588 256 et représentés par la formule :

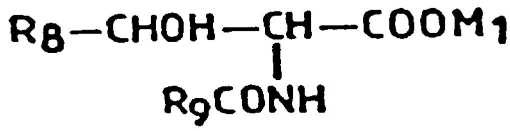

formule dans laquelle :

- . $R_8$ représente un radical alkyle ou alcényle en $C_7$-$C_{21}$;
- . $R_9$ représente un radical hydrocarboné, saturé ou insaturé en $C_7$-$C_{31}$, et
- . $M_1$ représente H, Na, K, $NH_4$, ou un ion ammonium substitué dérivé d'une amine,

(2)les composés anioniques, tels que les esters phosphoriques d'alcools gras, par exemple le dicétylphosphate et le dimyristylphosphate sous forme d'acides ou de sels alcalins ; l'acide heptylnonylbenzène sulfonique ; le sulfate de cholestérol acide et ses sels alcalins ; le phosphate de cholestérol acide et ses sels alcalins ; les lysolécithines ; les alkylsulfates, par exemple le cétyl sulfate de sodium, ; les gangliosides ;
(3)les lipides amphiphiles cationiques, tels que :

- les composés cationiques dérivés ammonium quaternaire répondant à la formule :

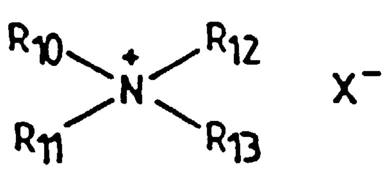

formule dans laquelle
$R_{10}$ et $R_{11}$ identiques ou différents, représentent des radicaux alkyle en $C_{12}$-$C_{20}$ et $R_{12}$ et $R_{13}$, identiques ou différents, des radicaux alkyle en $C_1$-$C_4$ ;

- les amines à longue chaîne et leurs dérivés ammonium quaternaire, les esters d'aminoalcools à longue chaîne et leurs sels et dérivés ammonium quaternaire ;

- des lipides polymérisables, comme ceux décrits par Ringsdorf et autres dans "Angewandte Chemie", vol 27, N°1, Janvier 1988, pages 129 et 137.

Le(s) lipide(s) amphiphile(s) utilisé(s) représente(nt), de préférence, de 0,5 à 25 % en poids par rapport au poids total de la composition.

Comme expliqué ci-dessus, la phase lipidique selon l'invention peut contenir également de façon connue des stérols, tels que les phytostérols et, plus particulièrement le cholestérol. La phase lipidique peut également éventuellement contenir des lipides chargés et ou des actifs liposolubles autres que le dérivé d'acide salicylique de formule (I) qui sont liposolubles tels que l'acide rétinoïque, les tocophérols, l'acide linoléique.

De façon connue la phase aqueuse E encapsulée et/ou la phase aqueuse D de dispersion peuvent contenir des actifs cosmétiques et/ou dermopharmaceutiques hydrosolubles tels que le glycérol, le sorbitol, l'érythrulose ou les antibiotiques.

Comme actifs cosmétiques et/ou dermopharmaceutiques, on peut citer les agents anti-oxydants, les agents anti-radicaux libres, les agents hydratants, les agents accélérateurs de bronzage, les agents dépigmentants, les agents de coloration de la peau, les agents liporégulateurs, les agents anti-vieillissement, les agents anti-UV, les agents kératolytiques, les agents émollients, les agents anti-inflammatoires, les agents rafraîchissants, les agents cicatrisants, les protecteurs vasculaires, les agents anti-bactériens, les agents antifongiques. On trouvera une liste de tels actifs dans la demande WO-A-92/08685.

Les vésicules ont avantageusement un diamètre moyen compris entre 100 et 500 nm.

La phase aqueuse D de dispersion selon l'invention peut, de façon connue, être constituée par de l'eau, ou un mélange d'eau et d'au moins un solvant miscible à l'eau tel que les alcools en $C_1$-$C_7$ et les polyols d'alkyle en $C_1$-$C_5$.

La phase aqueuse D de dispersion peut contenir, de façon connue, une dispersion de gouttelettes d'un liquide non miscible à l'eau, que les vésicules stabilisent. Par conséquent, en présence de vésicules de lipides amphiphiles ioniques et/ou de lipides amphiphiles non-ioniques, il n'est pas nécessaire d'introduire un émulsionnant usuel.

Selon la présente invention, le liquide non miscible à l'eau, qui peut être présent sous forme de dispersion dans la phase aqueuse D de dispersion, est constitué par tout liquide non miscible à l'eau connu de façon générale pour pouvoir être introduit dans la phase aqueuse de dispersion de vésicules de lipides ioniques ou non-ioniques ; il peut notamment être choisi dans le groupe formé par :

- une huile animale ou végétale formée par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tricaprocaprylate de glycérol, ou une huile végétale ou animale de formule $R_{15}$ $COOR_{16}$ formule dans laquelle $R_{15}$ représente le reste d'un acide gras supérieur comportant de 7 à 19 atomes de carbone et $R_{16}$ représente une chaîne hydrocarbonée ramifiée contenant de 3 à 20 atomes de carbone, par exemple l'huile de purcellin ;
- des huiles essentielles, naturelles ou synthétiques, telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, et de cade ;
- des hydrocarbures, tels que l'hexadécane et l'huile de paraffine ;
- des carbures halogénés, notamment des fluorocarbures tels que des fluoroamines, par exemple la perfluorotributylamine, des hydrocarbures fluorés, par exemple le perfluorodécahydronaphtalène, des fluoroesters et des fluoéthers ;
- des silicones, par exemple les polysiloxanes, les polydiméthylsiloxanes et les fluorosilicones ;
- des esters d'acide minéral et d'un alcool ;
- des éthers et des polyéthers ;

Le liquide non miscible à l'eau peut contenir un ou plusieurs actifs lipophiles.

La phase aqueuse D de la dispersion de vésicules selon l'invention peut aussi contenir , de façon connue, des additifs de formulation essentiellement nécessaires pour la formulation des compositions sous forme de lotion, crème ou sérum. Ces additifs sont, en particulier, pris dans le groupe formé par les gélifiants, les polymères, les conservateurs, les colorants, les opacifiants, les agents de contrôle du pH, les parfums. Parmi les gélifiants utilisables, on peut citer les dérivés de cellulose tels que l'hydroxyéthylcellulose; les dérivés d'algues tels que le satiagum; des gommes naturelles, telles que l'adragante, et des polymères synthétiques, en particulier les mélanges d'acides polycarboxyvinyliques commercialisés sous dénomination commerciale "CARBOPOL" par la société GOODRICH.

L'invention a également pour objet les dérivés de l'acide salicylique de formule (I) qui sont nouveaux.
Ce sont les composés de formule (I) :

I 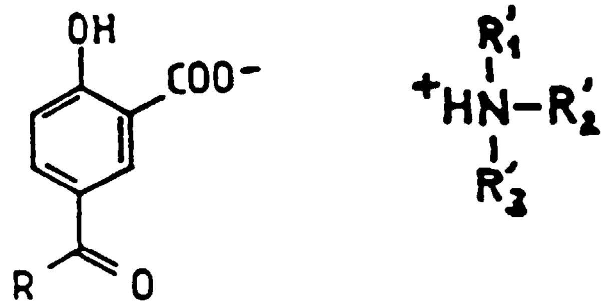

dans laquelle :

- R représente un radical alkyle linéaire ou ramifié en $C_{11}$-$C_{17}$, de préférence un radical alkyle linéaire en $C_{11}$,
- $R'_1$,$R'_2$, $R'_3$ identiques ou différents, représentent un radical alkyle ou hydroxyalkyle en $C_1$ à $C_{18}$, un des radicaux $R'_1$, $R'_2$ ou $R'_3$ pouvant être un radical benzyle, sous réserve que, lorsque R représente un radical alkyle en $C_{13}$ à $C_{17}$, $R'_1$ , $R'_2$ et $R'_3$ lorsqu'ils sont identiques ne représentent pas un radical hydroxyéthyle.

Ces composés sont avantageux, car comme expliqué ci-dessus, lorsqu'on les introduit dans la phase lipidique de la membrane de vésicules, ils servent à la fois de lipides chargés et d'actifs anti-acné.

Les composés de formule (I) sont préparés par salification de l'acide acyl-5 salicylique correspondant en présence d'une amine solubilisée dans un milieu alcoolique, tel que le méthanol, l'éthanol ou l'isopropanol. L'acide acyl-5 salicylique correspondant est obtenu par une réaction d'acylation du type FRIEDEL et CRAFTS entre un chlorure d'acide et l'ester méthylique de l'acide salicylique en présence d'un catalyseur tel que le chlorure d'aluminium anhydre. De telles réactions sont décrites particulièrement par OLAH, "FRIEDEL-CRAFTS and Related Reactions", Interscience Publishers, New york 1963-1964 ou par GORE, Chem. Rev. 55, 229-281 (1955).

Le schéma réactionnel est le suivant :

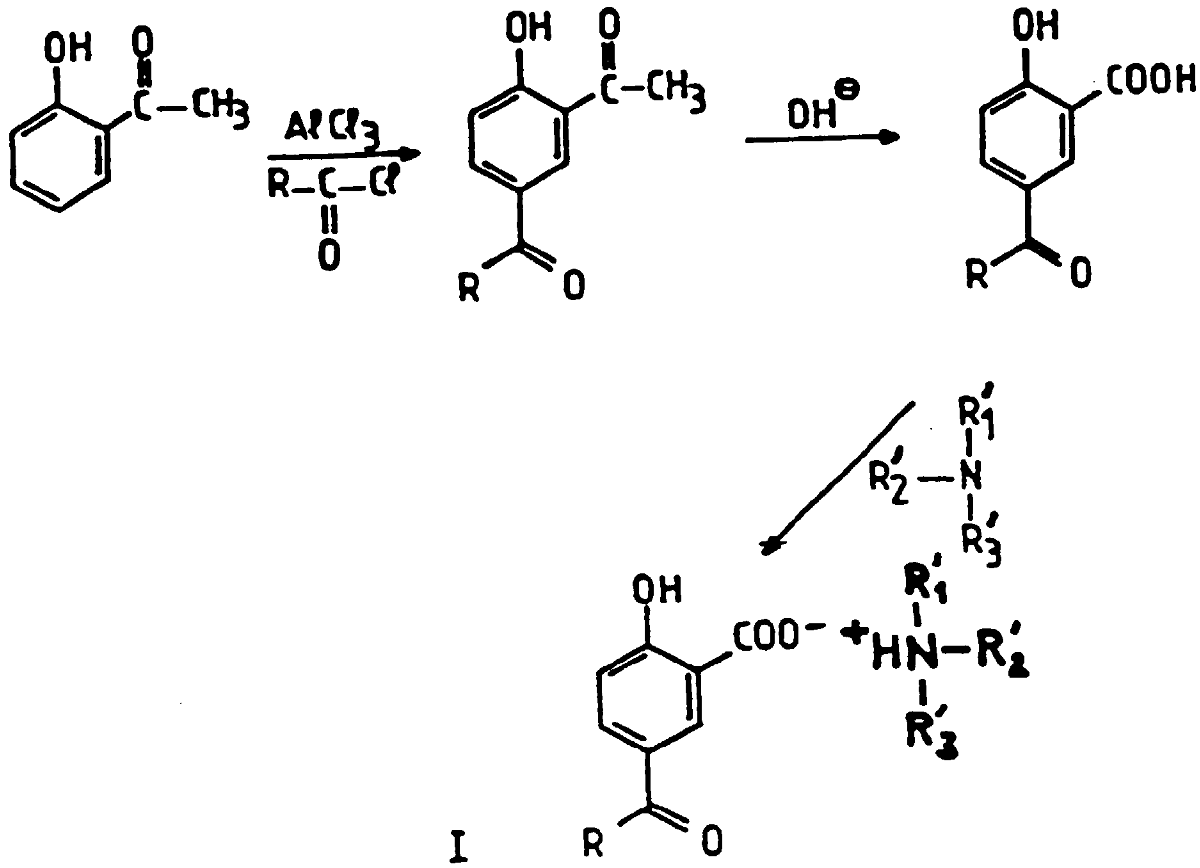


I

Les exemples donnés ci-après, à titre illustratif et nullement limitatif, permettront de mieux comprendre l'invention. Les exemples 1 à 4 ci-dessous concernent la préparation de dérivés d'acide salicylique de formule (I) selon l'invention.

EXEMPLE 1 : Préparation de n-dodécanoyl-5 salicylate de N, N, N-tri(hydroxy-2 propyl)ammonium.

Dans un bécher , on ajoute, sous agitation, 11,4 g (0,06 moles) de triisopropanolamine dans 100 ml d'isopropanol. On agite le milieu réactionnel et on refroidit entre 5 et 10°C. On ajoute ensuite 19,2 g (0,06 moles) d'acide n-dodécanoyl-5 salicylique. On agite pendant 30 min. à température ambiante puis on filtre le mélange limpide. Le filtrat est évaporé à sec. On obtient une huile, qui cristallise à température ambiante. On filtre sur fritté. La poudre blanche obtenue est séchée sous vide sur anhydride phosphorique. On obtient 29,1 g de produit, soit un rendement de 95%.

Les caractéristiques sont les suivantes :

Indice d'acide : 2,02-2,04 meq/g (théorie: 1,96)
Indice de basicité : 1,93-1,94 meq/g (théorie : 1,96)
Point de Kraft : 50°C

EXEMPLE 2: Préparation de n-dodécanoyl-5 salicylate de N-hydroxy tertiobutyl ammonium.

Le composé est préparé selon le même mode opératoire que dans l'exemple 1 en utilisant :

- 6,6 g (0,075 mole) d'amino-2 méthyl-2 propanol
- 24 g (0,075 mole) d'acide n-dodécanoyl-5 salicylique

On obtient 27,6 g d'une poudre blanche, soit un rendement de 90%.

L'analyse du produit obtenu est la suivante :

Indice d'acide : 2,58 meq/g (théorie : 2,44)
Indice de basicité: 2,36-2,38 meq/g (théorie : 2,44)

Point de Kraft : 47°C

<u>EXEMPLE 3</u> : Préparation de n-dodécanoyl-5 salicylate de N,N-diméthyl N-(hydroxy-2 éthyl) ammonium.

Le composé est préparé selon le même mode opératoire que dans l'exemple 1, en utilisant :

- 1,6 ml (0,0156 mole) de N, N-diméthyléthanolamine
- 5 g (0,0156 mole) d'acide n-dodécanoyl-5 salicylique

On obtient 6,1 g de produit sous forme de poudre blanche, soit un rendement de 95 %.
Le point de fusion du produit obtenu est de 58°C.
L'analyse élémentaire est la suivante :

| | C | H | N | O |
|---|---|---|---|---|
| % calculé | 67,48 | 9,53 | 3,42 | 19,56 |
| % trouvé | 67,44 | 9,60 | 3,34 | 19,51 |

Le spectre RMN $^{13}C$ est conforme à la structure attendue.

<u>EXEMPLE 4</u> : Préparation de n-dodécanoyl-5 salicylate de triéthylammonium.

Le composé est préparé selon le même mode opératoire que dans l'exemple 1, en utilisant :

- 6 g (0,06 mole) de triéthylamine
- 19,2 g (0,06 mole) d'acide n-dodécanoyl-5 salicylique

On obtient 22,7 g d'une poudre blanche, soit un rendement de 90%.
Les caractéristiques du produit obtenu sont les suivantes :

Indice d'acide : 2,48 - 2,49 meq/g (théorie : 2,38)
Indice de basicité : 2,26 meq/g (théorie : 2,38)

Les exemples 5 à 7 donnés ci-après sont des exemples de formulation de compositions contenant une dispersion en phase aqueuse de vésicules contenant dans la phase lipidique de la membrane des dérivés d'acide salicylique de formule (I).

<u>EXEMPLE 5 :</u> CREME DE SOIN DE LA PEAU

On réalise par fusion, à la température de 110°C, le mélange des produits suivants :
Composé amphiphile de formule :

$$C_{16}H_{33}\text{-O-}[C_3H_5(OH)\text{-O}]_{\bar{n}}\text{-H}$$

formule où $\bar{n}$ est une valeur statistique moyenne égale à 3 et où $-C_3H_5(OH)-O-$ représente les structures prises en mélange ou séparément :

$-CH_2\ CHOH\ CH_2O-$ ; $-CH_2-CH(CH_2OH)O-$ ; $-CH(CH_2OH)-CH_2O-$ .............. 3,6 g

Cholestérol .............................................................. 3,6 g

Composé de l'exemple 3.............................................. 0,8 g

Puis on ramène la température du mélange fondu à 90°C. On ajoute alors 20 g d'eau contenant 3 g de glycérine et on homogénéise le mélange obtenu à la température de 90°C. On ajoute alors 37,27 g d'eau à la température de 70°C, et on homogénéise le mélange obtenu à l'aide de l'ultra-disperseur "VIRTIS" pendant 4 minutes à la vitesse de 40000 t/mn.

On obtient ainsi une dispersion de vésicules lipidiques.

On ajoute alors 5 g d'eau contenant :

- 0,15 g de diazolidinyl urée vendu sous la dénomination "GERMAL II" par Société "SUTTON",
- et 0,05 g de sel dipotassique de l'acide éthylène diamino tétracétique.

On ajoute ensuite :

- 10 g de décaméthyl cyclo pentasiloxane vendue sous la dénomination "SILBIONE Huile 700 45 V5" par la Société "RHONE POULENC"
- 0,5 g de diméthicone vendue sous la dénomination "SILBIONE 747 V 300" par la Société "RHONE POULENC"
- 0,01 g de paraoxybenzoate de propyle

et on homogénéise à l'aide de l'ultra-disperseur "VIRTIS" pendant 4 minutes à 40000 t/mn.

On ajoute enfin :

- 0,42 g d'acide polyacrylique réticulé vendu sous la dénomination "CARBOPOL 940" par la Société "GOODRICH"
- 0,2 g de paraoxybenzoate de méthyle
- 15 g d'eau et
- 0,4 g de triéthanolamine

On obtient ainsi une crème blanche, épaisse. On observe après application de cette crème soir et matin à raison de 0,3 g sur le visage d'un sujet à peau acnéique, une régression nette de l'acné au bout de 3 semaines.

EXEMPLE 6 : CREME DE SOIN DE LA PEAU

Dans un ballon, on dissout les produits suivan(NR> :

- lécithine de soja hydrogénée vendue sous la dénomination "LECINOL S 10"par la Société "NIKKO" 2,4 g
- cholestérol 1,2 g
- composé de l'exemple 3 0,4 g

dans 50 ml d'un mélange solvant chloroforme/méthanol (1/1). On évapore ensuite le solvant à l'aide d'un évaporateur rotatif. Le mélange est alors hydraté à l'aide de 10 g d'eau à la température de 90°C et homogénéisé à l'aide d'une spatule puis transvasé dans un bécher. On ajoute alors, à la température de 70°C, 43,56 g d'eau. On homogénéise à l'aide d'un ultradisperseur "VIRTIS" pendant 4 minutes à la température de 70°C puis on revient à la température ambiante. On ajoute ensuite 5 g d'eau contenant 0,3 g d'imidazolidinyl-urée vendu sous la dénomination "GERMAL 115" par la Société "SUTTON" et 0,1 g de mélange de méthylchloroisothiazolinone et de méthylisothiazolinone vendu sous la dénomination "KATHON CG" par la Société "ROHM et HAAS". On ajoute ensuite 15 g d'huile de macadamia et on homogénéise à l'aide du disperseur Virtis pendant 4 minutes à 40000 t/mn.

On ajoute enfin :

- 0,42 g d'acide polyacrylique réticulé vendu sous la dénomination "CARBOPOL 940" par la Société "GOODRICH"
- 0,2 g de paraoxybenzoate de méthyle
- 15 g d'eau
- et 0,42 g de triéthanolamine

On obtient ainsi une crème blanche, épaisse. On observe, après application de cette crème soir et matin à raison de 0,2 g sur le visage d'un sujet à peau acnéique, une régression nette de l'acné au bout de 3 semaines.

EXEMPLE 7 : CREME DE SOIN DE LA PEAU

Selon le mode opératoire de l'exemple 5, on prépare une composition ayant la formulation suivante :

- composé amphiphile de l'exemple 5 2,7 g
- cholestérol 2,7 g
- dodécanoyl-5 salicylate de N, N-diméthyl N-(hydroxy-2-éthyl) ammonium (composé de l'exemple 3) 0,6 g
- glycérine 3 g
- sel disodique de l'acide éthylène diamino tétracétique 0,15g
- huile de tournesol 4 g
- butyl hydroxy toluène 0,05g
- huile de silicone vendue sous la dénomination "Volatil silicone 7158" par la Société "UNION CARBIDE" 3 g
- conservateurs qs
- eau distillée qsp 100 g
- acide polyacrylique réticulé vendu sous la dénomination "CARBOPOL 980" par la Société "GOODRICH" 0,8 g
- triéthanolamine 0,8 g

On obtient ainsi une crème blanche, épaisse. On observe, après application de cette crème soir et matin à raison de 0,2 g sur le visage d'un sujet à peau acnéique, une régression nette de l'acné au bout de 3 semaines.

EXEMPLE 8 : CREME DE SOIN DE LA PEAU

On réalise par fusion, à la température de 110°C, le mélange des produits suivants :
Composé amphiphile de formule :

$$C_{16}H_{33}-O-[C_3\ H_5(OH)-O]_{\bar{n}}-H$$

formule où $\bar{n}$ est une valeur statistique moyenne égale à 3 et où $-C_3\ H_5(OH)-O-$ représente les structures prises en mélange ou séparément :

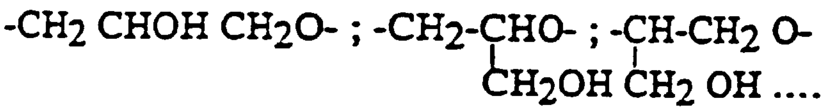
............ 3,6 g

Cholestérol ............ 3,6 g

Composé de l'exemple 1 ............ 0,8 g

Puis on ramène la température du mélange fondu à 90°C. On ajoute alors 20 g d'eau contenant 3 g de glycérine et on homogénéise le mélange obtenu à la température de 90°C. On ajoute alors 37,27 g d'eau à la température de 70°C, et on homogénéise le mélange obtenu à l'aide de l'ultra-disperseur "VIRTIS" pendant 4 minutes à la vitesse de 40000 t/mn.

On obtient ainsi une dispersion de vésicules lipidiques.

On ajoute alors 5 g d'eau contenant :

- 0,15 g de diazolidinyl urée vendu sous la dénomination "GERMAL II" par la Société "SUTTON",
- et 0,05 g de sel dipotassique de l'acide éthylène diamino tétracétique.

On ajoute ensuite :

- 10 g de décaméthyl cyclo pentasiloxane vendue sous la dénomination "SILBIONE Huile 700 45 V5" par la Société "RHONE POULENC"
- 0,5 g de diméthicone vendue sous la dénomination "SILBIONE 747 V 300" par la Société "RHONE POULENC"
- 0,01g de paraoxybenzoate de propyle

et on homogénéise à l'aide de l'ultra-disperseur "VIRTIS" pendant 4 minutes à 40000 t/mn.

On ajoute enfin :

- 0,42 g d'acide polyacrylique réticulé vendu sous la dénomination "CARBOPOL 940" par la Société "GOODRICH"

- 0,2 g de paraoxybenzoate de méthyle
- 15 g d'eau et
- 0,4 g de triéthanolamine

On obtient ainsi une crème blanche, épaisse. On observe après application de cette crème soir et matin à raison de 0,3 g sur le visage d'un sujet à peau acnéique, une régression nette de l'acné au bout de 3 semaines.

<u>EXEMPLE 9</u>: CREME DE SOIN DE LA PEAU

On réalise par fusion, à la température de 110°C, le mélange des produits suivants :
Composé amphiphile de formule :

$$C_{16}H_{33}\text{-}O\text{-}[C_3\ H_5(OH)\text{-}O]_{\bar{n}}\text{-}H$$

formule où $\bar{n}$ est une valeur statistique moyenne égale à 3 et où $-C_3\ H_5(OH)\text{-}O-$ représente les structures prises en mélange ou séparément :

$-CH_2\ CHOH\ CH_2O-$ ; $-CH_2\text{-}CHO-$ (avec $CH_2OH$ sur CH) ; $-CH\text{-}CH_2\ O-$ (avec $CH_2\ OH$ sur CH) ............. 3,6 g

Cholestérol ............................................................ 3,6 g

Composé de l'exemple 4 ............................................ 0,8 g

Puis on ramène la température du mélange fondu à 90°C. On ajoute alors 20 g d'eau contenant 3 g de glycérine et on homogénéise le mélange obtenu à la température de 90°C. On ajoute alors 37,27 g d'eau à la température de 70°C, et on homogénéise le mélange obtenu à l'aide de l'ultra-disperseur "VIRTIS" pendant 4 minutes à la vitesse de 40000 t/mn.

On obtient ainsi une dispersion de vésicules lipidiques.

On ajoute alors 5 g d'eau contenant :

- 0,15 g de diazolidinyl urée vendu sous la dénomination "GERMAL II" par la Société "SUTTON",
- et 0,05 g de sel dipotassique de l'acide éthylène diamino tétracétique.

On ajoute ensuite :

- 10 g de décaméthyl cyclo pentasiloxane vendue sous la dénomination "SILBIONE Huile 700 45 V5" par la Société "RHONE POULENC"
- 0,5 g de diméthicone vendue sous la dénomination "SILBIONE 747 V 300" par la Société "RHONE POULENC"
- 0,01 g de paraoxybenzoate de propyle

et on homogénéise à l'aide de l'ultra-disperseur "VIRTIS" pendant 4 minutes à 40000 t/mn.

On ajoute enfin :

- 0,42 g d'acide polyacrylique réticulé vendu sous la dénomination "CARBOPOL 940" par la Société "GOODRICH"
- 0,2 g de paraoxybenzoate de méthyle
- 15 g d'eau et
- 0,4 g de triéthanolamine

On obtient ainsi une crème blanche, épaisse. On observe après application de cette crème soir et matin à raison de 0,3 g sur le visage d'un sujet à peau acnéique, une régression nette de l'acné au bout de 3 semaines.

## Revendications

**1.** Composition pour le traitement de l'acné par application topique contenant une dispersion, dans une phase aqueuse D, de vésicules de lipide(s) amphiphile(s) constituées par une membrane de phase lipidique encapsulant une phase aqueuse E, la phase lipidique contenant un lipide chargé caractérisé par le fait que la phase lipidique contient au moins un dérivé d'acide salicylique de formule :

I 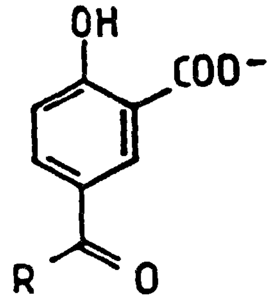 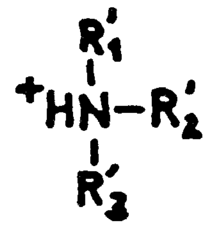

formule (I) dans laquelle

- R représente un radical alkyle linéaire ou ramifié en $C_{11}$-$C_{17}$,
- $R'_1$, $R'_2$, $R'_3$, identiques ou différents, représentent un radical alkyle ou hydroxyalkyle en $C_1$-$C_{18}$, un des radicaux $R'_1$, $R'_2$ ou $R'_3$ pouvant être un radical benzyle.

**2.** Composition selon la revendication 1, caractérisée par le fait que le dérivé de formule (I) est le n-dodécanoyl-5 salicylate de N, N-diméthyl N-(hydroxy-2 éthyl) ammonium.

**3.** Composition selon l'une des revendications 1 ou 2, caractérisée par le fait que la phase lipidique contient de 3 % à 10% en poids de composé(s) de formule (I).

**4.** Composition selon l'une des revendications 1 à 3, caractérisée par le fait que le(s) lipide(s) amphiphile(s) est (sont) choisi(s) dans le groupe formé par :

A/les lipides non-ioniques ci-après définis :

(1) les dérivés du glycérol, linéaires ou ramifiés, de formule :

$$R_oO-[C_3H_5(OH)O]_n-H$$

formule dans laquelle :

. $C_3H_5(OH)O$-
représente les structures suivantes, prises en mélange ou séparément :

$$-CH_2CHOHCH_2O-,$$

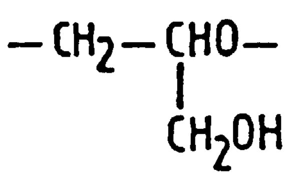 , 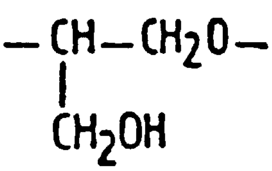

- n est une valeur statistique moyenne comprise entre 1 et 6 ou bien est une valeur réelle égale à 1 ou 2, auquel cas $C_3H_5(OH)O$ représente la structure $CH_2CHOH\text{-}CH_2O$ ;
- $R_o$ représente :

  (a)une chaîne aliphatique en $C_{12}$-$C_{30}$, linéaire ou ramifiée, saturée ou insaturée ; les radicaux hydrocarbonés des alcools de lanoline ; ou les restes d'alpha-diols à longue chaîne ;
  (b)un reste $R_1CO$, où $R_1$ est un radical aliphatique, linéaire ou ramifié, en $C_{11}$-$C_{29}$ ;
  (c)un reste :

$$R_2 - [OC_2H_3(R_3)] -$$

  où

- $R_2$ peut prendre la signification (a) ou (b) donnée pour $R_o$,
- $OC_2H_3(R_3)$-
  représente les structures suivantes, prises en mélange ou séparément :

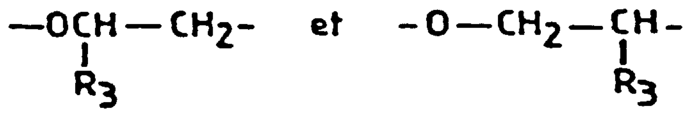

où $R_3$ prend la signification (a) donnée pour $R_o$ ;
(2)les éthers de polyglycérol, linéaires ou ramifiés, comportant deux chaînes grasses ;
(3)les diols à chaîne grasse ;
(4)les alcools gras, oxyéthylénés ou non, et les stérols , oxyéthylénés ou non ;
(5)les éthers et esters de polyols, oxyéthylénés ou non, l'enchaînement des oxydes d'éthylène pouvant être linéaire ou cyclique ;
(6)les glycolipides d'origine naturelle ou synthétique, les éthers et esters de mono ou polysaccharides ;
7/les hydroxyamides représentés par la formule :

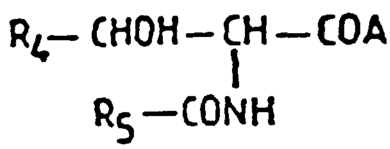

formule dans laquelle :

- $R_4$ désigne un radical alkyle ou alcényle en $C_7$-$C_{21}$ ;
- $R_5$ désigne un radical hydrocarboné, saturé ou insaturé, en $C_7$-$C_{31}$ ;
- COA désigne un groupement choisi parmi les deux groupements suivants :
- un reste

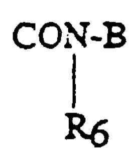

  où :

. B est un radical alkyle dérivé d'amines primaires ou secondaires, mono ou polyhydroxylées ; et
. $R_6$ désigne un atome d'hydrogène ou un radical méthyle, éthyle ou hydroxyéthyle ; et

- un reste -COOZ, où Z représente le reste d'un polyol en $C_3$-$C_7$

(8)les céramides naturels ou de synthèse ;
(9)les dihydroxyalkylanines, les amines grasses oxyéthylénées ;
(10)les dérivés du glycérol répondant à la formule :

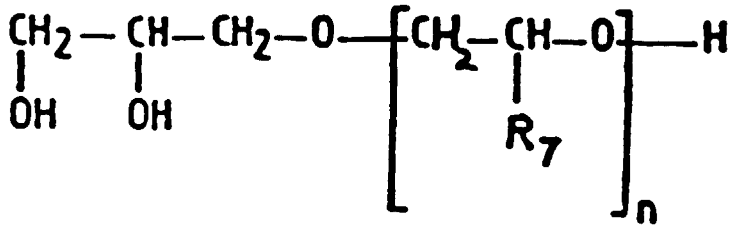

formule dans laquelle $R_7$ représente un radical alkyle linéaire en $C_{14}$-$C_{18}$ ou un groupement $CH_2A$ dans lequel A est $OR_{14}$, $R_{14}$ représentant un radical alkyle linéaire en $C_{10}$-$C_{18}$ et n représente une valeur statistique moyenne supérieure à 1 et au plus égale à 3 et, en outre, lorsque $R_7$ = $-CH_2A$, n peut également représenter une valeur réelle égale à 2.
(11)les esters de glucose de formule :

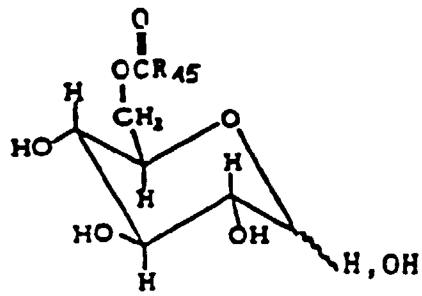

formule dans laquelle $R_{15}$ représente une chaîne hydrocarbonée linéaire en $C_9$-$C_{17}$, saturée ou insaturée.

B/les lipides amphiphiles ioniques ci-après définis :

(1) les lipides amphiphiles anioniques choisis parmi :

- les phospholipides naturels, les phospholipides modifiés par voie chimique ou enzymatique, et les phospholipides de synthèse ;
- les composés anioniques représentés par la formule :

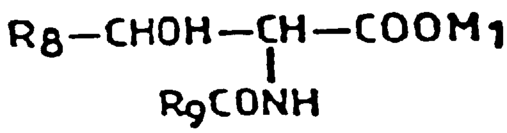

formule dans laquelle :

. $R_8$ représente un radical alkyle ou alcényle en $C_7$-$C_{21}$;

. $R_9$ représente un radical hydrocarboné, saturé ou insaturé, en $C_7$-$C_{31}$, et

. $M_1$ représente H, Na, K, $NH_4$, ou un ion ammonium substitué dérivé d'une amine ;

(2)les composés anioniques choisis parmi les esters phosphoriques d'alcools gras, ; l'acide heptylnonylbenzène sulfonique ; le sulfate de cholestérol acide et ses sels alcalins ;le phosphate de cholestérol acide et ses sels alcalins ; les lysolécithines ; les alkylsulfates, et les gangliosides ;
(3)les lipides amphiphiles cationiques choisis parmi :

- les composés cationiques dérivés ammonium quaternaire répondant à la formule :

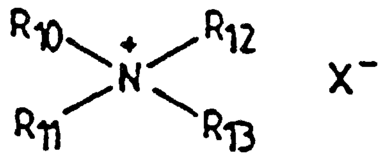

formule dans laquelle $R_{10}$ et $R_{11}$ identiques ou différents, représentent des radicaux alkyle en $C_{12}$-$C_{20}$ et $R_{12}$ et $R_{13}$, identiques ou différents, des radicaux alkyle en $C_1$-$C_4$ ;
- les amines à longue chaîne et leurs dérivés ammonium quaternaire, les esters d'aminoalcools à longue chaîne et leurs sels et dérivés ammonium quaternaire ;
- des lipides polymérisables,

**5.** Composition selon l'une des revendications 1 à 4, caractérisée par le fait que le(s) lipide(s) amphiphile(s) représente(nt) de 0,5 à 25 % en poids par rapport au poids total de la composition.

**6.** Composition selon l'une des revendications 1 à 5, caractérisée par le fait que la phase lipidique contient des stérols.

**7.** Composition selon la revendication 6, caractérisée par le fait que le stérol est du cholestérol.

**8.** Composition selon l'une des revendications 1 à 7 caractérisée par le fait que la phase lipidique contient un (des) lipide(s) chargé(s) autre(s) que le(s) dérivé(s) d'acide salicylique de formule (I).

**9.** Composition selon l'une des revendications 1 à 7, caractérisée par le fait que la phase lipidique contient des actifs cosmétiques et/ou dermopharmaceutiques liposolubles autres que le(s) dérivé(s) d'acide salicylique de formule (I).

**10.** Composition selon l'une des revendications 1 à 9 caractérisée par le fait que la phase aqueuse E des vésicules contient au moins un actif cosmétique et/ou dermopharmaceutique hydrosoluble.

**11.** Composition selon l'une des revendications 1 à 10 caractérisée par le fait que les vésicules ont un diamètre moyen compris entre 100 et 500 nm.

**12.** Composition selon l'une des revendications 1 à 11 caractérisée par le fait que la phase aqueuse D est constituée par de l'eau ou un mélange d'eau et d'au moins un solvant miscible à l'eau.

**13.** Composition selon l'une des revendications 1 à 12 caractérisée par le fait que la phase aqueuse D contient un actif cosmétique et/ou dermopharmaceutique hydrosoluble.

**14.** Composition selon l'une des revendications 1 à 13, caractérisée par le fait que la phase aqueuse D de dispersion contient une dispersion de gouttelettes d'un liquide non miscible à l'eau.

**15.** Composition selon l'une des revendications 1 à 13 caractérisée par le fait que la phase aqueuse D contient des additifs de formulation pris dans le groupe formé par les gélifiants, les polymères, les conservateurs, les colorants, les opacifiants, les agents de contrôle du pH et les parfums.

**16.** Dérivés de l'acide salicylique de formule :

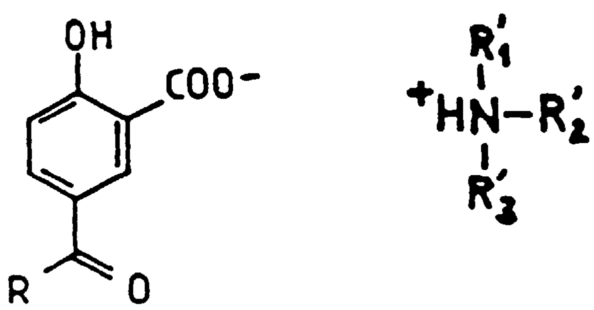

I

formule dans laquelle :

- R représente un radical alkyle linéaire ou ramifié en $C_{11}$-$C_{17}$;.
- $R'_1$, $R'_2$, $R'_3$, identiques ou différents, représentent un radical alkyle ou hydroxyalkyle en $C_1$-$C_{18}$, un des radicaux $R'_1$, $R'_2$, ou $R'_3$ pouvant être un radical benzyle, sous réserve que lorsque R représente un radical alkyle en $C_{13}$- $C_{17}$, $R'_1$, $R'_2$ et $R'_3$, lorsqu'ils sont identiques, ne représentent pas un radical hydroxyéthyle.

**Patentansprüche**

1. Mittel zur Behandlung von Akne durch topische Anwendung, enthaltend in einer wäßrigen Phase D eine Dispersion von Vesikeln aus amphiphilem(n) Lipid(en), die durch eine Membran der Lipidphase, welche eine wäßrige Phase E einkapselt, gebildet werden, wobei die Lipidphase ein geladenes Lipid enthält, dadurch gekennzeichnet, daß die Lipidphase wenigstens ein Salicylsäurederivat der Formel (I):

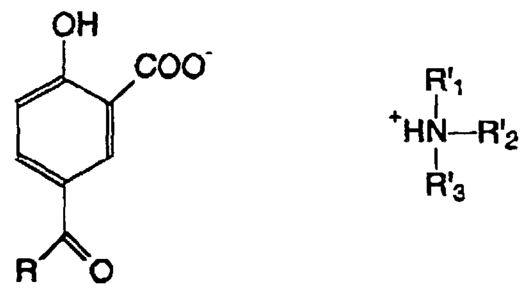

I

enthält, worin

- R für einen linearen oder verzweigten $C_{11}$-$C_{17}$-Alkylrest steht,
- $R'_1$, $R'_2$, $R'_3$, die gleich oder verschieden sein können, für einen $C_1$-$C_{18}$-Alkyl- oder -Hydroxyalkylrest stehen, wobei einer der Reste $R'_1$, $R'_2$ oder $R'_3$ ein Benzylrest sein kann.

2. Mittel nach Anspruch 1, dadurch gekennzeichnet, daß das Derivat der Formel (I) N,N-Dimethyl-N-(2-hydroxyethyl) ammonium-5-n-dodecanoylsalicylat ist.

3. Mittel nach einem der Ansprüche 1 oder 2, dadurch gekennzeichnet, daß die Lipidphase 3 bis 10 Gew.-% der Verbindung(en) der Formel (I) enthält.

4. Mittel nach einem der Ansprüche 1 bis 3, dadurch gekennzeichnet, daß das(die) amphiphile(n) Lipid(en) ausgewählt ist (sind) unter:

   (A) nachfolgend definierten nicht-ionischen Lipiden:

      (1) linearen oder verzweigten Glycerinderivaten der Formel:

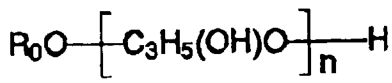

worin:

- $C_3H_5(OH)O$-
  für eine Kombination oder einzelne der folgenden Strukturen steht:

$$-CH_2CHOHCH_2O-,$$

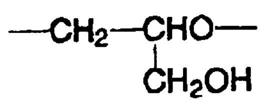 , 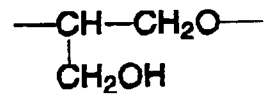

- n einen statistischen Mittelwert von 1 bis 6 oder einen tatsächlichen Wert von 1 oder 2 bedeutet, wobei in diesem Fall $C_3H_5(OH)O$ für die Struktur $CH_2CHOH\text{-}CH_2O$ steht;
- $R_o$ für:

  (a) eine gesättigte oder ungesättigte, lineare oder verzweigte, aliphatische $C_{12}$-$C_{30}$-Kette; Kohlenwasserstoffreste von Lanolinalkoholen; oder langkettige alpha-Diolreste;
  (b) einen Rest $R_1CO$, worin $R_1$ für einen linearen oder verzweigten aliphatischen $C_{11}$-$C_{29}$-Rest steht;
  (c) einen Rest:

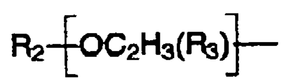

  steht,
  worin

- $R_2$ die für $R_o$ angegebene Bedeutung (a) oder (b) annehmen kann,
- $OC_2H_3(R_3)$- für eine Kombination oder einzelne der folgenden Strukturen steht:

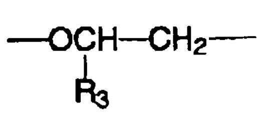 und 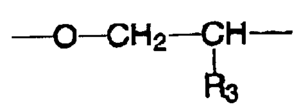

  worin $R_3$ die für $R_o$ angegebene Bedeutung (a) besitzt;

(2) linearen oder verzweigten Polyglycerinethern, die zwei Fettketten tragen;
(3) Diolen mit Fettkette;
(4) gegebenenfalls ethoxylierten Fettalkoholen und gegebenenfalls ethoxylierten Sterolen;
(5) gegebenenfalls oxyethylenierten Polyolethern und -estern, wobei die Ethylenoxidketten linear oder cyclisch sein können;
(6) Glycolipiden natürlichen oder synthetischen Ursprungs, Ethern und Estern von Mono- oder Polysacchariden;
(7) Hydroxamiden der Formel:

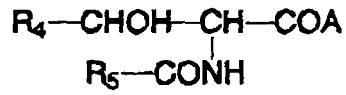

worin:

- $R_4$ für einen $C_7$-$C_{21}$-Alkyl- oder Alkenylrest steht;
- $R_5$ für einen gesättigten oder ungesättigten $C_7$-$C_{31}$-Kohlenwasserstoffrest steht;
- COA für eine Gruppe steht, die ausgewählt ist unter den folgenden zwei Gruppen:
  - einem Rest

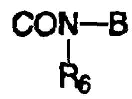

worin:

. B für einen mono- oder polyhydroxylierten, von primären oder sekundären Aminen abgeleiteten Alkylrest steht; und

. $R_6$ für ein Wasserstoffatom oder einen Methyl-, Ethyl-oder Hydroxyethylrest steht; und

  - einem Rest -COOZ, worin Z für einen $C_3$-$C_7$-Polyolrest steht;

(8) natürlichen oder synthetischen Ceramiden;
(9) Dihydroxyalkylaminen, ethoxylierten Fettaminen;
(10) Glycerinderivaten der Formel:

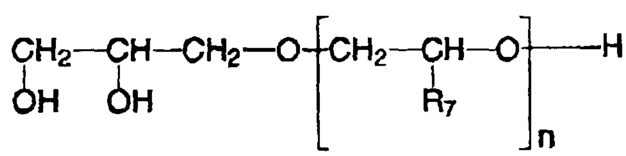

worin $R_7$ für einen linearen $C_{14}$-$C_{18}$-Alkylrest oder für eine $CH_2A$-Gruppe steht, worin A für $OR_{14}$ steht, wobei $R_{14}$ für einen linearen $C_{10}$-$C_{18}$-Alkylrest steht und n für einen statistischen Mittelwert von mehr als 1 und höchstens gleich 3 steht und außerdem, falls $R_7$ = $-CH_2A$ ist, n auch einen tatsächlichen Wert von 2 bedeuten kann;
(11) Glucoseestern der Formel:

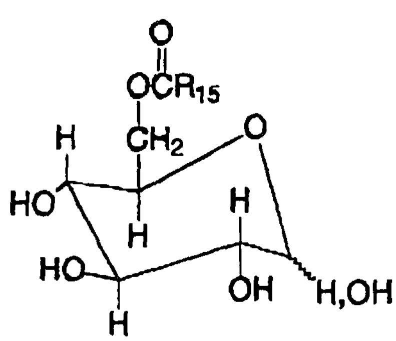

worin $R_{15}$ für eine gesättigte oder ungesättigte, lineare $C_9$-$C_{17}$-Kohlenwasserstoffkette steht.

(B) den nachfolgend definierten ionischen amphiphilen Lipiden:

(1) anionischen amphiphilen Lipiden, die ausgewählt sind unter:

- natürlichen Phospholipiden, auf chemischem oder enzymatischem Weg modifizierten Phospholipiden, und synthetischen Phospholipiden;
- anionischen Verbindungen der Formel:

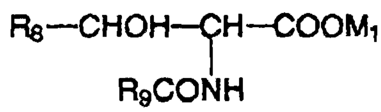

worin:

. $R_8$ für einen $C_7$-$C_{21}$-Alkyl- oder Alkenylrest steht;
. $R_9$ für einen gesättigten oder ungesättigten $C_7$-$C_{31}$-Kohlenwasserstoffrest steht, und
. $M_1$ für H, Na, K, $NH_4$ oder ein von einem Amin abgeleitetes substituiertes Ammoniumion steht;

(2) anionischen Verbindungen, die ausgewählt sind unter Phosphorsäureestern von Fettalkoholen; Heptylnonylbenzolsulfonsäure; Cholesterinsäuresulfat und dessen Alkalisalzen; Cholesterinsäurephosphat und dessen Alkalisalzen; Lysolecithinen; Alkylsulfaten und Gangliosiden;
(3) kationischen amphiphilen Lipiden, die ausgewählt sind unter:

- von quaternären Ammoniumionen abgeleiteten kationischen Verbindungen der Formel:

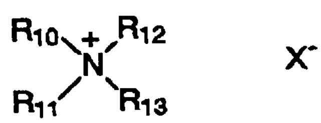

worin $R_{10}$ und $R_{11}$, die gleich oder verschieden sein können, für $C_{12}$-$C_{20}$-Alkylreste stehen und $R_{12}$ und $R_{13}$, die gleich oder verschieden sein können, für $C_1$-$C_4$-Alkylreste stehen;
- langkettigen Aminen und deren quaternären Ammoniumderivaten, Estern langkettiger Aminoalkohole und deren Salzen und quaternären Ammoniumderivaten;
- polymerisierbaren Lipiden.

**5.** Mittel nach einem der Ansprüche 1 bis 4, dadurch gekennzeichnet, daß das (die) amphiphile(n) Lipid(en) 0,5 bis 25 Gew.-%, bezogen auf das Gesamtgewicht des Mittels, ausmachen.

**6.** Mittel nach einem der Ansprüche 1 bis 5, dadurch gekennzeichnet, daß die Lipidphase Sterole enthält.

**7.** Mittel nach Anspruch 6, dadurch gekennzeichnet, daß das Sterol Cholesterin ist.

**8.** Mittel nach einem der Ansprüche 1 bis 7, dadurch gekennzeichnet, daß die Lipidphase ein anderes geladenes Lipid (andere geladene Lipide) als das (die) Salicylsäurederivat(e) der Formel (I) enthält.

**9.** Mittel nach einem der Ansprüche 1 bis 7, dadurch gekennzeichnet, daß die Lipidphase andere fettlösliche, kosmetische und/oder dermopharmazeutische Wirkstoffe als das (die) Salicylsäurederivat(e) der Formel (I) enthält.

**10.** Mittel nach einem der Anprüche 1 bis 9, dadurch gekennzeichnet, daß die wäßrige Phase E der Vesikel wenigstens einen wasserlöslichen kosmetischen und/oder dermopharmazeutischen Wirkstoff enthält.

**11.** Mittel nach einem der Ansprüche 1 bis 10, dadurch gekennzeichnet, daß die Vesikel einen mittleren Durchmesser

von 100 bis 500 nm aufweisen.

**12.** Mittel nach einem der Ansprüche 1 bis 11, dadurch gekennzeichnet, daß die wäßrige Phase D aus Wasser oder einer Mischung von Wasser mit wenigstens einem mit Wasser mischbarem Lösungsmittel gebildet wird.

**13.** Mittel nach einem der Ansprüche 1 bis 12, dadurch gekennzeichnet, daß die wäßrige Phase D einen wasserlöslichen, kosmetischen und/oder dermopharmazeutischen Wirkstoff enthält.

**14.** Mittel nach einem der Ansprüche 1 bis 13, dadurch gekennzeichnet, daß die wäßrige Dispersionsphase D eine Dispersion von Tröpfchen einer nicht mit Wasser mischbaren Flüssigkeit enthält.

**15.** Mittel nach einem der Ansprüche 1 bis 13, dadurch gekennzeichnet, daß die wäßrige Phase D Formulierungshilfsmittel enthält, die ausgewählt sind unter Geliermitteln, Polymeren, Konservierungsmitteln, Farbstoffen, Trübungsmitteln, Mitteln zur Kontrolle des pH-Werts und Parfums.

**16.** Salicylsäurederivate der Formel:

I 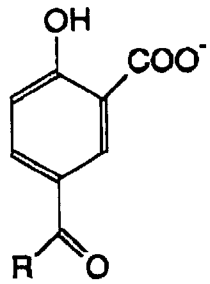 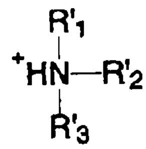

worin:

- R für einen linearen oder verzweigten $C_{11}$-$C_{17}$-Alkylrest steht;
- $R'_1$, $R'_2$, $R'_3$, die gleich oder verschieden sein können, für einen $C_1$-$C_{18}$-Alkyl- oder -Hydroxyalkylrest stehen, wobei einer der Reste $R'_1$, $R'_2$ oder $R'_3$ ein Benzylrest sein kann, mit der Maßgabe, daß, falls R für einen $C_{13}$-$C_{17}$-Alkylrest steht, $R'_1$, $R'_2$ und $R'_3$, falls sie identisch sind, nicht für einen Hydroxyethylrest stehen.

**Claims**

**1.** Composition for the treatment of acne by topical application containing a dispersion, in an aqueous phase D, of vesicles of amphiphilic lipid(s) consisting of a lipid phase membrane encapsulating an aqueous phase E, the lipid phase containing a charged lipid characterized by the fact that the lipid phase contains at least one salicylic acid derivative of formula:

I 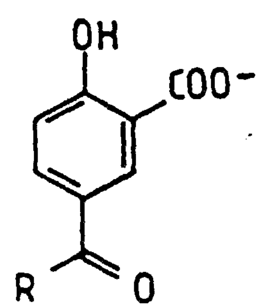 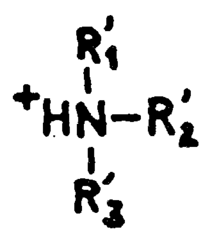

in which formula (I):

- R represents a linear or branched $C_{11}$-$C_{17}$ alkyl radical,
- $R'_1$, $R'_2$ and $R'_3$, which are identical or different, represent a $C_1$-$C_{18}$ alkyl or hydroxyalkyl radical, it being possible for one of the radicals $R'_1$, $R'_2$ or $R'_3$ to be a benzyl radical.

**2.** Composition according to Claim 1, characterized by the fact that the derivative of formula (I) is N,N-dimethyl-N-(2-hydroxyethyl)ammonium 5-n-dodecanoylsalicylate.

**3.** Composition according to one of Claims 1 or 2, characterized by the fact that the lipid phase contains 3% to 10% by weight of compound(s) of formula (I).

**4.** Composition according to one of Claims 1 to 3, characterized by the fact that the amphiphilic lipid(s) is (are) chosen from the group consisting of:

A/ the non-ionic lipids defined below:

(1) the linear or branched glycerol derivatives of formula:

$$R_oO - [C_3H_5(OH)O]_n - H$$

in which formula:

$$C_3H_5(OH)O-$$

represents the following structures taken together or separately:

$$-CH_2CHOHCH_2O-,$$

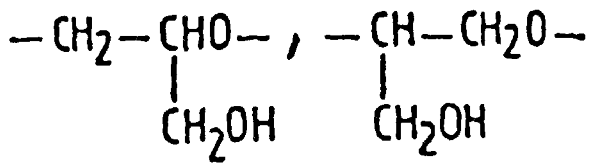

. n is a mean statistical value between 1 and 6 or alternatively n is a real value equal to 1 or 2, in which case $C_3H_5(OH)O$ represents the structure

$$CH_2CHOH-CH_2O$$

. $R_o$ represents:

(a) a saturated or unsaturated linear or branched $C_{12}$-$C_{30}$ aliphatic chain; hydrocarbon radicals of lanolin alcohols; or long-chain alpha-diol residues;
(b) a residue $R_1CO$, where $R_1$ is a linear or branched $C_{11}$-$C_{29}$ aliphatic radical;
(c) a residue:

$$R_2-[OC_2H_3(R_3)]-$$

where

. $R_2$ may have the meaning (a) or (b) given for $R_o$;

$$-OC_2H_3(R_3)-$$

represents the following structures, taken together or separately,

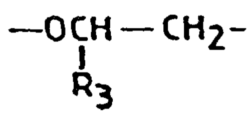

and

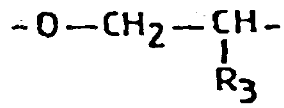

where

- $R_3$ has the meaning (a) given for $R_o$;

(2) linear or branched polyglycerol ethers containing two fatty chains;
(3) fatty chain diols;
(4) oxyethylenated or non-oxyethylenated fatty alcohols, and oxyethylenated or non-oxyethylenated sterols;
(5) oxyethylenated or non-oxyethylenated polyol ethers and esters, it being possible for the chain of ethylene oxides to be linear or cyclic;
(6) glycolipids or natural or synthetic origin, mono- or polysaccharide ethers and esters;
(7) the hydroxyamided represented by the formula:

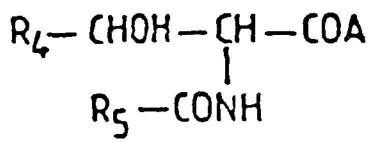

in which formula:

- $R_4$ designates a $C_7$-$C_{21}$ alkyl or alkenyl radical;
- $R_5$ designates a saturated or unsaturated $C_7$-$C_{31}$ hydrocarbon radical;
- COA designates a group chosen from the following two groups:

. a residue

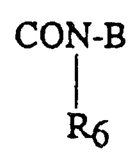

where:
. B is an alkyl radical derived from mono- or polyhydroxylated primary or secondary amines; and

. $R_6$ designates a hydrogen atom or a methyl, ethyl or hydroxyethyl radical; and
. a residue -COOZ where Z represents a $C_3$-$C_7$ polyol residue;

(8) natural or synthetic ceramides;
(9) dihydroxyalkylamines, oxyethylenated fatty amines;
(10) the glycerol derivatives corresponding to the formula:

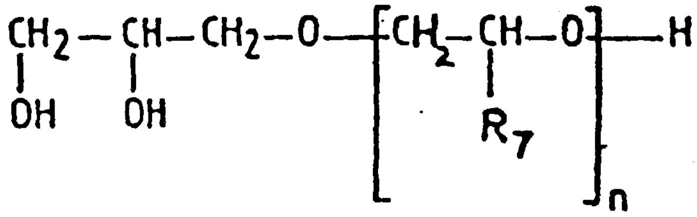

in which formula $R_7$ represents a linear $C_{14}$-$C_{18}$ alkyl radical or a group $-CH_2A$ in which A is $OR_{14}$, $R_{14}$ representing a linear $C_{10}$-$C_{18}$, alkyl radical, and n represents a mean statistical value greater than 1 and not more than 3 and, in addition, when $R_7 = -CH_2A$, n may also represent a real value equal to 2.
(11) Glucose esters of formula:

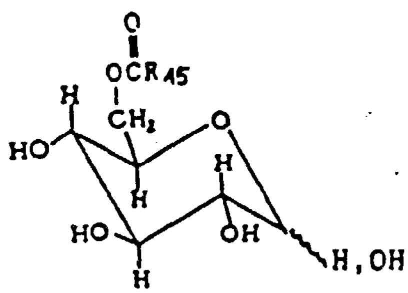

in which formula $R_{15}$ represents a saturated or unsaturated linear $C_9$-$C_{17}$ hydrocarbon chain.

B) The ionic amphiphilic lipids defined below:

(1) anionic amphiphilic lipids such as:

. natural phospholipids, phospholipids modified chemically or enzymatically, and synthetic phospholipids;
. anionic compounds represented by the formula:

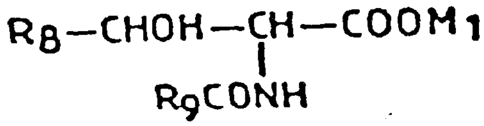

in which formula:
. $R_8$ represents a $C_7$-$C_{21}$ alkyl or alkenyl radical;
. $R_9$ represents a saturated or unsaturated $C_7$-$C_{31}$ hydrocarbon radical, and
. $M_1$ represents H, Na, K, $NH_4$, or a substituted ammonium ion derived from an amine,

(2) anionic compounds chosen from phosphoric esters of fatty alcohols,; heptyl nonyl benzene sulphonic acid; acid cholesterol sulphate and its alkali metal salts; acid cholesterol phosphate and its alkali metal salts; lysolecithins; alkyl sulphates, and gangliosides;
(3) cationic amphiphilic lipids chosen from:

- quaternary ammonium-derived cationic compounds corresponding to the formula:

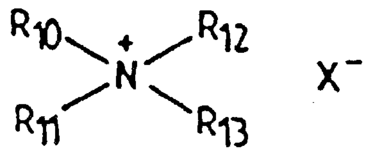

  in which formula:
  $R_{10}$ and $R_{11}$, which are identical or different, represent $C_{12}$-$C_{20}$ alkyl radicals and $R_{12}$ and $R_{13}$, which are identical or different, $C_1$-$C_4$ alkyl radicals;
- long chain amines and their quaternary ammonium derivatives, esters of long-chain amino alcohols and their salts and quaternary ammonium derivatives;
- polymerizable lipids.

**5.** Composition according to one of Claims 1 to 4, characterized by the fact that the amphiphilic lipid(s) represents (represent) 0.5 to 25% by weight relative to the total weight of the composition.

**6.** Composition according to one of Claims 1 to 5, characterized by the fact that the lipid phase contains sterols.

**7.** Composition according to Claim 6, characterized by the fact that the sterol is cholesterol.

**8.** Composition according to one of Claims 1 to 7, characterized by the fact that the lipid phase contains (a) charged lipid(s) other than the salicylic acid derivative(s) of formula (I).

**9.** Composition according to one of Claims 1 to 7, characterized by the fact that the lipid phase contains fat-soluble cosmetic and/or dermopharmaceutical active ingredients other than the salicylic acid derivative(s) of formula (I).

**10.** Composition according to one of Claims 1 to 9, characterized by the fact that the aqueous phase E of the vesicles contains at least one water-soluble cosmetic and/or dermopharmaceutical active ingredient.

**11.** Composition according to one of Claims 1 to 10, characterized by the fact that the vesicles have a mean diameter of between 100 and 500 nm.

**12.** Composition according to one of Claims 1 to 11, characterized by the fact that the aqueous phase D consists of water or a mixture of water and at least one water-miscible solvent.

**13.** Composition according to one of Claims 1 to 12, characterized by the fact that the aqueous phase D contains a water-soluble cosmetic and/or dermopharmaceutical active ingredient.

**14.** Composition according to one of Claims 1 to 13, characterized by the fact that the aqueous dispersion phase D contains a dispersion of droplets of a water-immiscible liquid.

**15.** Composition according to one of Claims 1 to 13, characterized by the fact that the aqueous phase D contains formulation additives chosen from the group consisting of gelling agents, polymers, preservatives, colorants, opacifying agents, pH-regulating agents and perfumes.

**16.** Salicylic acid derivatives of formula:

I 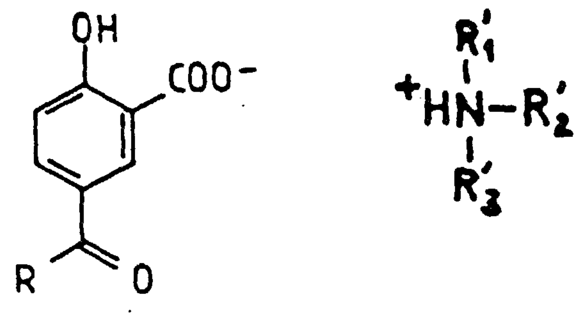

in which formula:

- R represents a linear or branched $C_{11}$-$C_{17}$ alkyl radical;
- $R'_1$, $R'_2$, $R'_3$, which are identical or different, represent a $C_1$-$C_{18}$ alkyl or hydroxyalkyl radical, it being possible for one of the radicals $R'_1$, $R'_2$, or $R'_3$ to be a benzyl radical, provided that when R represents a $C_{13}$-$C_{17}$ alkyl radical, $R'_1$, $R'_2$ and $R'_3$, when they are identical, do not represent a hydroxyethyl radical.